Europäisches Patentamt

European Patent Office

Office européen des brevets

(11) Veröffentlichungsnummer: **0 496 206 B1**

(12) **EUROPÄISCHE PATENTSCHRIFT**

(45) Veröffentlichungstag der Patentschrift: **05.04.95**

(51) Int. Cl.6: **C07C 17/00**, C07C 25/02

(21) Anmeldenummer: **92100191.3**

(22) Anmeldetag: **08.01.92**

(54) **Verfahren zur Umsetzung von polyhalogenierten Aromaten mit monohalogenierten oder nicht halogenierten Aromaten.**

(30) Priorität: **19.01.91 DE 4101528**

(43) Veröffentlichungstag der Anmeldung:
**29.07.92 Patentblatt 92/31**

(45) Bekanntmachung des Hinweises auf die
Patenterteilung:
**05.04.95 Patentblatt 95/14**

(84) Benannte Vertragsstaaten:
**BE DE FR GB IT NL**

(56) Entgegenhaltungen:
**EP-A- 0 256 479**

**CHEMICAL ABSTRACTS, Band 113, Nr. 15, 8.
Oktober 1990, Columbus, Ohio, US, abstract
no. 131713j, Seite 603; K. SHINODA et al:
"Transchlorination of aromatic polychlorides"**

**CHEMISTRY LETTERS, Bd. 10, Nr. 10, 1987,
Tokoy, JP, Seiten 2051 - 2052; K.SHINODA:
"Transchlorination of o-Dichlorobenzene
and Benzene into Chlorobenzene"**

(73) Patentinhaber: **BAYER AG**

**D-51368 Leverkusen (DE)**

(72) Erfinder: **Mais, Franz-Josef, Dr.
Gustav-Poensgen-Strasse 23
W-4000 Düsseldorf 1 (DE)**
Erfinder: **Fiege, Helmut, Dr.
Walter-Flex-Strasse 23
W-5090 Leverkusen 1 (DE)**

**Beschreibung**

Die vorliegende Erfindung betrifft ein Verfahren, bei dem polyhalogenierte Aromaten mit monohalogenierten oder nicht halogenierten Aromaten zur Umsetzung gebracht werden mit dem Ziel, das Halogen des polyhalogenierten Aromaten auf den monohalogenierten oder nicht halogenierten Aromaten Zu übertragen und gleichzeitig die Wasserstoffatome des monohalogenierten oder nicht halogenierten Aromaten auf den polyhalogenierten Aromaten zu übertragen.

Aus der japanischen Anmeldung JP-01 311-032 ist die Umsetzung von polychlorierten Aromaten wie z.B. 1,2-Dichlorbenzol oder 1,2,4-Trichlorbenzol mit Benzol bekannt. Als Katalysator setzt man Aktivkohle ein, die mit Palladiumchlorid ($PdCl_2$) und einem Seltenerdemetallchlorid wie z.B. $CeCl_3$ beladen ist. Bei dieser Umsetzung entstehen im allgemeinen Gemische aus den beiden Ausgangsprodukten wie z.B. 1,2-Dichlorbenzol und Benzol mit dem Produkt der Chlorübertragungen Chlorbenzol sowie mit den Isomerisierungsprodukten des polychlorierten Ausgangsmaterials 1,3- und 1,4-Dichlorbenzol.

Aus der allgemeinen chemischen Literatur (Chem. Lett. 1987, 2051) ist weiterhin die Umsetzung von 1,2-Dichlorbenzol mit Benzol im Gegenwart von mit Chloriden des Iridiums, Rhodiums oder Osmiums beladener Aktivkohle bekannt. Diese Katalysatoren zeigen allerdings wesentlich schlechtere Wirkung bei der Chlorübertragung als die mit Palladiumchlorid/Seltenerdechlorid beladenen Aktivkohlen.

Aus der EP 256 479 ist weiterhin die Umsetzung von Polyjodbenzol wie z.B. 1,4-Dijodbenzol mit Benzol bekannt. Als Katalysatoren werden Zeolithe des Typs X oder Y eingesetzt, die mit Thallium oder Seltenerdemetallen ausgetauscht sein können Das Reaktionsprodukt ist ein Gemisch aus dem im Überschuß eingesetzten Benzol und Jodbenzol.

Es wurde nun ein Verfahren zur Umsetzung von polyhalogenierten Aromaten mit monohalogenierten oder nicht halogenierten Aromaten gefunden, bei dem das Halogen der polyhalogenierten Aromaten auf die monohalogenierten oder nicht halogenierten Aromaten und gleichzeitig der Wasserstoff der monohalogenierten oder nicht halogenierten Aromaten auf die polyhalogenierten Aromaten in Gegenwart eines Katalysators übertragen wird, dadurch gekennzeichnet, daß man einen Katalysator einsetzt, der Ruthenium als Element oder in Form von Verbindungen und gegebenenfalls ein oder mehrere Metalle oder Metallverbindungen aus der I. und II. Nebengruppe des Periodensystems enthält.

Als polyhalogenierte Aromaten können erfindungsgemäß polyhalogenierte Benzole, polyhalogenierte Naphthaline oder polyhalogenierte Biphenyle eingesetzt werden. Diese polyhalogenierten Einsatzstoffe tragen erfindungsgemäß mindestens 2 Halogenatome bei Verwendung von nichthalogenierten und mindestens 3 Halogenatome bei Verwendung von monohalogenierten Aromaten bis zur jeweiligen maximalen Sättigung, d.h. im Benzolsystem 2 bis 6 Halogenatome, im Naphthalinsystem 2 bis 8 Halogenatome und im Biphenylsystem 2 bis 10 Halogenatome.

Als Halogen in den polyhalogenierten Einsatzstoffen ist erfindungsgemäß Chlor, Brom oder Jod einsetzbar, bevorzugt Chlor oder Brom, ganz besonders bevorzugt Chlor.

Die polyhalogenierten Aromaten können durch ein einheitliches Halogen polysubstituiert sein. Es ist jedoch auch möglich, daß der polyhalogenierte Aromat durch zwei oder auch drei verschiedene Halogene polysubstituiert ist. Erfindungsgemäß bevorzugt sind jedoch durch ein Halogen einheitlich polysubstituierte Aromaten.

Als Beispiel für erfindungsgemäß einsetzbare polyhalogenierte Aromaten seien die Folgenden genannt, ohne die Erfindung jedoch auf diese einzuschränken:

1,2-Dichlorbenzol,

1,3-Dichlorbenzol,

1,4-Dichlorbenzol,

1,2,4-Trichlorbenzol,

1,2,3-Trichlorbenzol,

1,3,5-Trichlorbenzol,

1,2,3,4-Tetrachlorbenzol,

1,2,4,5-Tetrachlorbenzol,

Pentachlorbenzol,

Hexachlorbenzol,

stellungsisomere Dibrombenzole,

stellungsisomere Tribrombenzole,

stellungsisomere Tetrabrombenzole,

stellungsisomere Dijodbenzole,

stellungsisomere Dichlornaphthaline,

stellungsisomere Tri- und Tetrachlornaphthaline,

2

stellungsisomere Di-, Tri- und Tetrachlorbiphenyle,
stellungsisomere Di-, Tri- und Tetrabrombiphenyle.

Es ist erfindungsgemäß bevorzugt, Gemische der polyhalogenierten Aromaten einzusetzen, beispielsweise seien genannt:

Ein Gemisch aus wechselnden Anteilen aus Tri- und Tetrachlorbenzolen mit kleineren Anteilen an Penta- und Hexachlorbenzol.

Ein Gemisch chlorierter Naphthaline mit dem mittleren Chloriergrad von beispielsweise 4 bis 6.

Ein Gemisch chlorierter Biphenyle mit dem Chloriergrad von beispielsweise 3 bis 6.

Es ist erfindungsgemäß weiterhin möglich, auch Gemische von polyhalogenierten Benzolen und polyhalogenierten Naphthalinen oder von polyhalogenierten Benzolen und polyhalogenierten Biphenylen usw. einzusetzen, dies ist jedoch nicht bevorzugt.

Als monohalogenierten oder nicht halogenierten Aromaten kann man erfindungsgemäß Halogenbenzol oder Benzol einsetzen. Als Halogen in dem monohalogenierten Aromaten ist erfindungsgemäß Fluor, Chlor, Brom oder Jod einsetzbar. Erfindungsgemäß bevorzugt ist es, den nicht halogenierten Aromaten Benzol einzusetzen.

Das Einsatzverhältnis der polyhalogenierten Aromaten zu den monohalogenierten oder nicht halogenierten Aromaten ist im Rahmen der vorliegenden Erfindung prinzipiell beliebig. Im Interesse einer praktischen Nutzung ist es jedoch wenig sinnvoll, den monohalogenierten Aromaten in einem molaren Unterschuß einzusetzen. Es ist daher erfindungsgemäß bevorzugt, den monohalogenierten oder nicht halogenierten Aromaten in einem molaren Überschuß einzusetzen.

Die erfindungsgemäß einsetzbaren Katalysatoren enthalten Ruthenium als Element oder in Form von Verbindungen. Zur Durchführung eines kontinuierlichen industriellen Prozesses ist es notwendig, das Ruthenium oder dessen Verbindungen in eine handhabbare Form zu überführen. Es ist daher erfindungsgemäß bevorzugt, das Ruthenium oder dessen Verbindungen auf ein Trägermaterial aufzubringen. Es ist weiterhin erfindungsgemäß bevorzugt, daß dieses Trägermaterial eine gegebenenfalls gekörnte Aktivkohle ist.

Die Herstellung der rutheniumbeladenen Aktivkohle kann z.B. dadurch erfolgen, daß man eine z.B. gekörnte Aktivkohle mit einer beispielsweise wäßrigen Rutheniumsalzlösung tränkt und anschließend trocknet. Die so gewonnenen trockenen, mit Rutheniumverbindungen beladenen Katalysatoren können dann gegebenenfalls durch Waschen mit weiteren Lösungen wie z.B. wäßriger Kochsalzlösung, wäßriger Ammoniaklösung, wäßriger Hydrazinlösung, verdünnter wäßriger Säure usw. weiter modifiziert werden.

Es ist weiterhin möglich, die so beladenen Kontakte erneut mit einer z.B. wäßrigen Metallösung zu tränken und erneut zu trocknen. Man erhält auf diese Weise einen Katalysator, der neben dem Rutheniumsalz noch ein oder mehrere Salze von weiteren sogenannten Co-Metallsalzen enthält. Es ist erfindungsgemäß ganz besonders bevorzugt, Katalysatoren aus Aktivkohle, die mit Rutheniumsalzen und einem oder mehreren Co-Metallsalzen beladen sind, einzusetzen.

Als Rutheniumsalz kann man alle einfachen löslichen Rutheniumsalze wie z.B. die Halogenide einsetzen, besonders bevorzugt ist Rutheniumchlorid. Als Co-Metallsalze kann man erfindungsgemäß Salze von Elementen der I. und II. Nebengruppe, d.h. z.B. Kupfer, Silber, Gold, Zink, Quecksilber oder Cadium einsetzen, ganz besonders bevorzugt sind Salze von Kupfer und Silber.

Das molare Verhältnis von Rutheniumsalz zu Co-Metallsalz oder zu der Summe der Co-Metallsalze kann in einem Bereich von 1:10 bis 10:1 variiert werden, bevorzugt ist ein Molverhältnisbereich von 2:1 bis 1:2.

Das erfindungsgemäße Verfahren wird in gasförmiger Phase durchgeführt, wobei der feste heterogene Katalysator in einer technisch üblichen Anordnung wie z.B. als Festbett oder Wirbelbett vorliegen kann.

Neben den gasförmigen bzw. dampfförmigen Einsatzstoffen und deren gas- bzw. dampfförmigen Reaktionsprodukten kann im Reaktionsraum noch ein unter den Reaktionsbedingungen inertes Gas oder ein inerter Dampf zu Verdünnung vorhanden sein. Als Beispiele ohne einschränkende Wirkung seien genannt: Stickstoff, Argon oder Wasserdampf.

Das erfindungsgemäße Verfahren kann bei Normaldruck, erniedrigtem Druck oder erhöhtem Druck durchgeführt werden. Bevorzugt ist Normaldruck oder erhöhter Druck.

Die erfindungsgemäßen Reaktionstemperaturen sind in einem weiten Bereich variierbar. Nach unten ist die Reaktionstemperatur dadurch begrenzt, daß bei dem gewählten Reaktionsdruck alle Einsatzstoffe und deren Reaktionsprodukte in gasförmiger Phase vorliegen sollen. Nach oben ist die Temperatur dadurch begrenzt, daß bei zu hoher Temperatur Nebenreaktionen wie z.B. Spaltungsreaktionen des Aromaten oder Polymerbildung eintreten können, die somit zur Desaktivierung des Katalysators führen können. Im allgemeinen wählt man erfindungsgemäß die Reaktionstemperatur zwischen 200°C und 600°C, bevorzugt ist ein Bereich von 300 bis 500°C.

Eine beispielhafte Ausführungsform des erfindungsgemäßen Verfahrens ist z.B. die folgende.

Man stellt einen Katalysator z.B. aus einer auf Holzkohle basierenden, mit Dampf aktivierten und extra gut gewaschenen Kornaktivkohle her, der 5 g Ruthenium pro Liter als Rutheniumchlorid enthält. Nach dem Trocknen wird dieser Katalysator mit einer Co-Metallsalzlösung (z.B: $CuCl_2$-Lösung oder $AgNO_3$-Lösung) getränkt und erneut getrocknet. Der Katalysator, der nun die zwei Salze in einem Molverhältnis von z.B. 1:1 enthält, wird in ein Reaktionsrohr gegeben mit einem Verhältnis des Durchmessers des Reaktionsrohres zum Durchmesser der Katalysatorkörnung von 20 zu 1 bis 50 zu 1. Anschließend wird er 2 h bei der gewünschten Temperatur von z.B. 400°C unter einem leichten Inertgasstrom von z.B. Stickstoff ausgeheizt. Danach wird eine Mischung eines polyhalogenierten Aromaten mit einem monohalogenierten oder nicht halogenierten Aromaten, z.B. isomere Trichlorbenzole mit Benzol, gasförmig aus einer Vorverdampferzone und einer Vorheizzone über den auf die gewünschte Temperatur von 400°C vorgeheizten Katalysator geleitet. Gegebenenfalls kann man zusätzlich inertes Gas, z.B. Stickstoff, einblasen. Das gasförmige Produktgemisch wird durch eine kurze Nachheizzone geführt und in einer gekühlten Produktfalle kondensiert.

Das erfindungsgemäße Verfahren ermöglicht die Übertragung des Halogens eines polyhalogenierten Aromaten auf einen mono- oder nicht halogenierten Aromaten in Gegenwart von rutheniumhaltigen Katalysatoren. Daß gerade diese rutheniumhaltigen Katalysatoren bei der Übertragung des Halogens gute Wirkung zeigen, muß nach dem Stand der Technik als äußerst überraschend gelten. Bisher waren lediglich mit u.a. Palladiumchlorid beladene Aktivkohlen für eine ähnliche Chlorübertragung bekannt. Aus der allgemeinen chemischen Literatur (Chem. Lett. 1987, 2051; Chem. Lett. 1989, 1265) ist weiterhin zu entnehmen, daß andere Edelmetallchloride wirkungslos oder wirkungsschwach sind.

Es ist weiterhin ausgesprochen überraschend, daß eine deutliche Aktivitätssteigerung der rutheniumhaltigen Katalysatoren durch die Anwesenheit von zusätzlichen Co-Metallen der I. und II. Nebengruppe des Periodensystems, wie z.B. Kupfer- oder Silberverbindungen, eintritt. Diese Tatsache ist aus dem bekannten chemischen Wissen nicht herleitbar.

Die erfindungsgemäß geschaffene Möglichkeit, das Halogen eines z.B. polychlorierten Aromaten auf beispielsweise einen nicht halogenierten zu übertragen, ist äußerst wertvoll für z.B. die Entsorgung von hochchlorierten Abfallisomerengemischen der Benzolchlorierung oder für z.B. die Enthalogenierung und damit Entsorgung von polyhalogenierten Naphthalinen oder Biphenylgemischen. Gleichzeitig kann man bei der Entchlorierung von polychlorierten Benzolen durch Umsetzung mit Benzol noch Chlorbenzol und gegebenenfalls Dichlorbenzole als Wertprodukte gewinnen.

Die folgenden Beispiele sollen das erfindungsgemäße Verfahren verdeutlichen:

Beispiele

Beispiel 1

Herstellung eines Katalysators(Ru/Cu-1)

1 Liter einer trockenen, auf Holzkohle basierenden, gut gewaschenen und mit Dampf aktivierten Kornaktivkohle (Korngröße 1-3 mm Durchmesser, Schüttgewicht ca. 400 g/l) wurde mit soviel einer kommerziell erhältichen wäßrigen Rutheniumchloridlösung, die im wesentlichen Ruthenium der Oxidationsstufe + 3 enthielt, getränkt, daß der Katalysator nach dem Trocknen bei 100°C im Vakuum 5,00 g Ruthenium pro Liter als Chlorid enthielt. Der trockene Katalysator wurde mit einer Lösung von 8,40 g $CuCl_2$ • 2 $H_2O$ in 170 ml Wasser getränkt und erneut bei 100°C im Vakuum getrocknet. Der trockene Katalysator enthielt nun 5,00 g Ru/l und 3,14 g Cu/l im wesentlichen als Chloride. Das Molverhältnis der Elemente Ruthenium und Kupfer betrug 1:1. Das Schüttgewicht des trockenen Katalysators betrug ca. 420 g/l.

Beispiel 2

Herstellung eines Katalysators (Ru/Cu-2)

Das Verfahren des Beispiels 1 wurde wiederholt, nur daß statt einer auf Holzkohle basierenden Kornaktivkohle eine auf Torf basierende gut gewaschene, dampfaktivierte trockene Formaktivkohle (Strangpreßlinge, Länge: 2 bis 5 mm, Durchmesser: ca. 0,8 mm, Schüttgewicht: ca. 390 g/l) eingesetzt wurde. Der fertige trockene Katalysator enthielt 5,00 g Ru/l und 3,14 g Cu/l, entsprechend einem Molverhältnis der beiden Elemente von ca. 1:1. Das Schüttgewicht des trockenen Katalysators betrug ca. 410 g/l.

4

EP 0 496 206 B1

Beispiel 3

Herstellung eines Katalysators (Ru/Ag-1)

1 Liter der Kornaktivkohle des Beispiels 1 wurde wie dort beschrieben mit soviel Rutheniumchloridlösung getränkt und getrocknet, daß 5,00 g Ru/l resultierten. Dieser trockene Katalysator wurde mit einer Lösung von 8,40 g AgNO₃ in 180 ml Wasser getränkt und erneut bei 100°C im Vakuum getrocknet. Dieser trockene Kontakt wurde nun mit 3,30 l einer 1%igen wäßrigen Kochsalzlösung gewaschen und erneut bei 100°C im Vakuum getrocknet. Er enthielt nun 5,00 g Ru/l und 5,34 g Ag/l. Das entsprach einem Molverhältnis von Ruthenium zu Silber von ca. 1:1. Das Schüttgewicht des trockenen Katalysators betrug ca. 420 g/l.

Beispiel 4

Herstellung eines Katalysators (Ru/Ag-2)

Das Verfahren des Beispiels 3 wurde wiederholt, nur daß statt der dortigen Aktivkohle die in Beispiel 2 beschriebene, auf Torf basierende Formkohle eingesetzt wurde. Der trockene, fertige Kontakt enthielt nun 5,00 g Ru/l und 5,34 g Ag/l entsprechend einem Molverhältnis der beiden Elemente von ca. 1:1. Das Schüttgewicht des trockenen Katalysators betrug ca. 419 g/l.

Beispiele 5 bis 8

In einem senkrecht stehenden Reaktionsrohr wurden jeweils 150 ml der in den Beispielen 1 bis 4 beschriebenen Katalysatoren eingefüllt. Man erhielt ein Katalysatorfestbett von ca. 21 cm Höhe, das von außen beheizt wurde. Zunächst wurde das Katalysatorbett 2 Stunden unter einem Sticksstoffstrom von 20 l/h bei 400°C ausgeheizt. Dann wurde mit einer Geschwindigkeit von 15,0 g/h ein Gemisch aus 13,5 g 1,2,4-Trichlorbenzol und 76,5 g Benzol gasförmig, aus einem Vorverdampfer, und auf 400°C vorgeheizt über das Katalysatorbett bei 400°C geleitet. Dabei wurde zusätzlch langsam Stickstoff mit einer Rate von 5-7 l/h eingeblasen. Nach dem Ende der Zugabe, d.h. nach 6 h, wurde zusätzlich noch 1 h unter einem Stickstoffstrom von 5-7 l/h nachgeheizt. Die Abgase wurden in einer Kühlfalle kondensiert Nach zwei, vier und sieben Stunden wurde diese Kühlfalle gewechselt und das Kondensat mittels einer geeichten GC-Methode analysiert, so daß die GC-%-Angaben Gewichtsprozenten entsprechen. Die Ergebnisse der vier Versuche finden sich in den Tabellen 1 bis 4.

Vergleichbeispiel 9

(Katalysator nach JP-01/311-032)

Die in Beispiel 2 beschriebene Formaktivkohle wurde wie im Beispiel 1 beschrieben mit soviel einer kommerziell erhältlichen wäßrigen Lösung von PdCl₂ getränkt, daß der Katalysator nach dem Trocknen 5,00 g Pd/l enthält. Der trockene Katalysator wurde nun mit einer Lösung von 17,53 g CeCl₃ • 7 H₂O in 170 ml Wasser getränkt und bei 100°C im Vakuum getrocknet. Das Schüttgewicht betrug danach ca. 425 g/l. Dieser Katalysator wurde wie in den Beispielen 5-8 beschrieben nun 2 h bei 400°C unter Stickstoff ausgeheizt. Anschließend wurden analog den Beispielen 5-8 13,5 g 1,2,4-Trichlorbenzol und 76,5 g Benzol durch das Festbett geleitet. Das Kondensat wurde analog den Beispielen 5-8 aufgefangen und analysiert. Das Ergebnis findet sich ebenfalls in den Tabellen 1 bis 4.

5

Ergebnis der Beispiele 5-8 und des Vergleichsbeispiel 9 in den Tabellen 1-4

| Beispiele | Katalysator | Katalysator-menge | Einsatzprodukte | Dosier-zeit | Nachheiz-zeit | Tempera-tur |
|---|---|---|---|---|---|---|
| 5 | Ru/Cu-1 aus Beispiel 1 | 150 ml | 13,5 g 1,2,4-Trichlorbenzol + 76,5 g Benzol | 6 h | 1 h | 400° C |
| 6 | Ru/Cu-2 aus Beispiel 2 | 150 ml | 13,5 g 1,2,4-Trichlorbenzol + 76,5 g Benzol | 6 h | 1 h | 400° C |
| 7 | Ru/Ag-1 aus Beispiel 3 | 150 ml | 13,5 g 1,2,4-Trichlorbenzol + 76,5 Benzol | 6 h | 1 h | 400° C |
| 8 | Ru/Ag-2 aus Beispiel 4 | 150 ml | 13,5 g 1,2,4-Trichlorbenzol + 76,5 g Benzol | 6 h | 1 h | 400° C |
| Ver-gleichs-beispiel 9 | Pd/Ce | 150 ml | 13,5 g 1,2,4-Trichlorbenzol + 76,5 g Benzol | 6 h | 1 h | 400° C |

Tabelle 1: Versuchsbedingungen der Beispiele 5-8 und des Vergleichsbeispieles 9.

EP 0 496 206 B1

| Beispiel | Benzol | Chlor-benzol | 1,2-Di-chlor-benzol | 1,3-Di-chlor-benzol | 1,4-Di-chlor-benzol | 1,2,3-Tri-chlorbenzol | 1,2,4-Tri-chlorbenzol | Auswaage g | Auswaage % Einsatz |
|---|---|---|---|---|---|---|---|---|---|
| 5 | 89,49 % | 10,50 % | - | 0,15 % | - | - | - | 27,83 | 92,77 |
| 6 | 90,50 % | 9,50 % | - | - | - | - | - | 26,97 | 89,90 |
| 7 | 87,88 % | 12,12 % | - | - | - | - | - | 26,13 | 87,10 |
| 8 | 88,42 % | 11,58 % | - | - | - | - | - | 26,30 | 87,66 |
| Ver-gleichs-bei-spiel 9 | 90,19 % | 9,38 % | 0,08 % | 0,27 % | 0,08 % | - | - | 25,90 | 86,33 |

Tabelle 2: Zusammensetzung und Auswaage des Kondensats der Beispiele 5-8 und des Vsergleichsbeispiel 9 für die Versuchszeit von 0 - 2 h.

EP 0 496 206 B1

| Beispiel | Benzol | Chlor-benzol | 1,2-Di-chlor-benzol | 1,3-Di-chlor-benzol | 1,4-Di-chlor-benzol | 1,2,3-Tri-chlorbenzol | 1,2,4-Tri-chlorbenzol | Auswaage g | Auswaage % Einsatz |
|---|---|---|---|---|---|---|---|---|---|
| 5 | 90,89 % | 9,11 % | - | - | - | - | - | 26,93 | 89,78 |
| 6 | 90,96 % | 9,04 % | - | - | - | - | - | 25,87 | 86,23 |
| 7 | 86,31 % | 13,12 % | 0,47 % | 0,10 % | - | - | - | 29,03 | 96,78 |
| 8 | 86,48 % | 12,99 % | 0,37 % | 0,13 % | 0,03 % | - | - | 28,30 | 94,33 |
| Ver-gleichs-bei-spiel 9 | 87,78 % | 8,47 % | 1,05 % | 1,20 % | 1,09 % | - | - | 27,50 | 91,67 |

Tabelle 3: Zusammensetzung und Auswaage des Kondensats der Beispiele 5-8 und des Vergleichsbeispieles 9 für die Versuchszeit von 2 - 4 h.

EP 0 496 206 B1

| Beispiel | Benzol | Chlor-benzol | 1,2-Di-chlor-benzol | 1,3-Di-chlor-benzol | 1,4-Di-chlor-benzol | 1,2,3-Tri-chlorbenzol | 1,2,4-Tri-chlorbenzol | Auswaage g | Auswaage % Einsatz |
|---|---|---|---|---|---|---|---|---|---|
| 5 | 89,75 % | 9,17 % | 0,19 % | 0,50 % | 0,39 % | - | - | 28,03 | 93,44 |
| 6 | 90,69 % | 8,00 % | 0,10 % | 0,70 % | 0,51 % | - | - | 28,37 | 94,57 |
| 7 | 85,48 % | 11,01 % | 2,56 % | 0,48 % | 0,47 % | - | - | 29,63 | 98,78 |
| 8 | 87,16 % | 8,51 % | 2,51 % | 0,82 % | 0,71 % | - | 0,29 % | 29,50 | 98,33 |
| Ver-gleichs-bei-spiel 9 | 86,47 % | 5,78 % | 1,65 % | 2,61 % | 1,68 % | - | 1,81 % | 29,60 | 98,67 |

Tabelle 4: Zusammensetzung und Auswaage des Kondensats der Beispiele 5 - 8 und des Vergleichsbeispiel 9 für die Versuchszeit von 4 - 7 h (Ende der Nachlaufzeit).

Beispiel 10

Das Verfahren des Beispiels 8 wurde wiederholt, nur daß statt der dortigen Mischung aus 13,5 g 1,2,4-Trichlorbenzol und 76,5 g Benzol hier eine Mischung aus 16,4 g 1,2-Dichlorbenzol und 73,6 g Benzol eingesetzt wurde. Das Ergebnis findet man in den Tabellen 5 bis 8.

Beispiel 11

Das Verfahren des Beispiels 8 wurde wiederholt, nur daß statt der dortigen Mischung aus 13,5 g 1,2,4-Trichlorbenzol und 76,5 g Benzol hier eine Mischung aus 6,57 g 1,2,4,5-Tetrachlorbenzol und 83,43 g Benzol über den Katalysator geleitet wurde. Das Ergebnis findet man in den Tabellen 5 bis 8.

Beispiel 12

Das Verfahren des Beispiels 8 wurde wiederholt, nur daß statt der dortigen Einsatzmischung ein Gemisch aus 3,87 g Hexachlorbenzol und 86,13 g Benzol eingesetzt wurde. Das Ergebnis findet man ebenfalls in den Tabellen 5 bis 8.

Beispiel 13

Das Verfahren des Beispiels 8 wurde wiederholt, nur daß statt der dortigen Einsatzmischung ein Gemisch aus 9,81 g 1,2,4-Trichlorbenzol und 80,19 g Chlorbenzol eingesetzt wurde. Das Versuchsergebnis findet sich in den Tabellen 5 bis 8.

Ergebnis der Versuche 10 bis 13 in den Tabellen 5 bis 8

| Beispiele | Katalysator | Katalysator-menge | Einsatzprodukte | Dosier-zeit | Nachheiz-zeit | Tempera-tur |
|---|---|---|---|---|---|---|
| 10 | Ru/Ag-2 aus Beispiel 4 | 150 ml | 16,4 g 1,2-Dichlorbenzol + 73,6 g Benzol | 6 h | 1 h | $400^0$ C |
| 11 | Ru/Ag-2 aus Beispiel 4 | 150 ml | 6,57g 1,2,4,5-Tetrachlorbenzol+ 83,43 g Benzol | 6 h | 1 h | $400^0$ C |
| 12 | Ru/Ag-2 aus Beispiel 4 | 150 ml | 3,87 g Hexachlorbenzol + 86,13 g Benzol | 6 h | 1 h | $400^0$ C |
| 13 | Ru/Ag-2 aus Beispiel 4 | 150 ml | 9,81 g 1,2,4-Trichlorbenzol + 80,19 g Chlorbenzol | 6 h | 1 h | $400^0$ C |

Tabelle 5: Versuchsbedingungen der Beispiele 10 - 13.

EP 0 496 206 B1

| Beispiel | Benzol | Chlor-benzol | 1,2-Di-chlor-benzol | 1,3-Di-chlor-benzol | 1,4-Di-chlor-benzol | 1,2,3-Tri-chlorbenzol | 1,2,4-Tri-chlorbenzol | Auswaage g | Auswaage % Einsatz |
|---|---|---|---|---|---|---|---|---|---|
| 10 | 86,30 % | 13,61 % | 0,09 % | - | - | - | - | 27,13 | 90,43 |
| 11 | 93,99 % | 6,01 % | - | - | - | - | - | 27,30 | 91,00 |
| 12 | 93,68 % | 6,32 % | - | - | - | - | - | 27,20 | 90,67 |
| 13 | 6,36 % | 70,96 % | 2,36 % | 14,03 % | 5,73 % | 0,12 % | 0,44 % | 21,60 | 72,00 |

Tabelle 6: Zusammensetzung und Auswaage des Kondensats der Beispiele 10 - 13 für die Versuchszeit von 0 - 2 h.

| Beispiel | Benzol | Chlor-benzol | 1,2-Di-chlor-benzol | 1,3-Di-chlor-benzol | 1,4-Di-chlor-benzol | 1,2,3-Tri-chlorbenzol | 1,2,4-Tri-chlorbenzol | Auswaage g | Auswaage % Einsatz |
|---|---|---|---|---|---|---|---|---|---|
| 10 | 84,44 % | 13,64 % | 1,30 % | 0,40 % | 0,22 % | - | - | 29,33 | 97,77 |
| 11 | 94,05 % | 5,95 % | - | - | - | - | - | 29,00 | 96,67 |
| 12 | 93,98 % | 6,02 % | - | - | - | - | - | 28,30 | 94,33 |
| 13 | 4,42 % | 75,79 % | 2,32 % | 12,67 % | 4,56 % | 0,06 % | 0,18 % | 25,70 | 85,67 |

Tabelle 7: Zusammensetzung und Auswaage des Kondensats der Beispiele 10 - 13 für die Versuchszeit von 2 bis 4 h.

13

| Beispiel | Benzol | Chlor-benzol | 1,2-Di-chlorbenzol | 1,3-Di-chlorbenzol | 1,4-Di-chlorbenzol | 1,2,3-Tri-chlorbenzol | 1,2,4-Tri-chlorbenzol | Auswaage g | Auswaage % Einsatz |
|---|---|---|---|---|---|---|---|---|---|
| 10 | 83,50 % | 10,98 % | 4,33 % | 0,65 % | 0,37 % | - | 0,17 % | 30,83 | 102,78 |
| 11 | 94,37 % | 5,63 % | - | - | - | - | - | 27,90 | 93,00 |
| 12 | 94,02 % | 5,92 % | 0,06 % | - | - | - | - | 27,50 | 91,67 |
| 13 | 2,44 % | 81,84 % | 1,89 % | 9,67 % | 3,56 % | 0,08 % | 0,88 % | 33,05 | 110,17 |

Tabelle 8: Zusammensetzung und Auswaage des Kondensats der Beispiele 10 - 13 für die Versuchszeit von 4 - 7 h (Ende der Nachlaufzeit).

Beispiel 14

Das Verfahren des Beispieles 8 wurde wiederholt, nur daß statt des dortigen Einsatzgemisches aus 13,5 g 1,2,4-Trichlorbenzol und 76,5 g Benzol ein Einsatzgemisch aus 18,00 g 1,3,5-Tribrombenzol und 72,00 g Benzol über den Katalysator geleitet wurden.

| | |
|---|---|
| Versuchsbedingungen : | 150 ml Katalysator Ru/Ag-2 (Beispiel 4) Dosierzeit 6 h, Nachlaufzeit: 1 h, Temperatur: 400°C |
| Zusammensetzung 0 - 2 h: | 90,51 % Benzol, 5,81 % Chlorbenzol, 3,68 % Brombenzol; |
| Auswaage: | 27,63 g, 92,10 % vom Einsatz |
| Zusammensetzung 2 - 4 h: | 89,23 % Benzol, 0,43 % Chlorbenzol, 10,34 % Brombenzol; |
| Auswaage: | 28,34 g 94,47 % vom Einsatz |
| Zusammensetzung 4 - 7 h: | 89,71 % Benzol, 10,22 % Brombenzol, 0,07 % 1,3-Dibrombenzol; |
| Auswaage: | 28,61 g, 95,37 % vom Einsatz |

Beispiel 15

Das Verfahren des Beispiels 7 wurde wiederholt, nur daß statt bei 400°C bei einer Reaktionstemperatur von 350°C gearbeitet wurde. Das Ergebnis findet man in den Tabellen 9-12.

Beispiel 16

Das Verfahren des Beispiels 7 wurde wiederholt, nur daß statt bei 400°C bei einer Reaktionstemperatur von 450°C gearbeitet wurde. Das Ergebnis findet sich in Tabellen 9-12.

Ergebnis der Versuche 15 und 16 in Tabellen 9 bis 12

| Beispiele | Katalysator | Katalysator-menge | Einsatzprodukte | Dosier-zeit | Nachheiz-zeit | Tempera-tur |
|---|---|---|---|---|---|---|
| 15 | Ru/Ag-1 aus Beispiel 3 | 150 ml | 13,5 g 1,2,4-Trichlorbenzol + 76,5 g Benzol | 6 h | 1 h | 350° C |
| 16 | Ru/Ag-1 aus Beispiel 3 | 150 ml | 13,5 g 1,2,4-Trichlorbenzol + 76,5 g Benzol | 6 h | 1 h | 450° C |

Tabelle 9: Versuchsbedingungen der Beispiele 15 - 16.

EP 0 496 206 B1

| Beispiel | Benzol | Chlor-benzol | 1,2-Di-chlor-benzol | 1,3-Di-chlor-benzol | 1,4-Di-chlor-benzol | 1,2,3-Tri-chlorbenzol | 1,2,4-Tri-chlorbenzol | Auswaage g | Auswaage % Einsatz |
|---|---|---|---|---|---|---|---|---|---|
| 15 | 88,36 % | 11,64 % | - | - | - | - | - | 26,67 | 88,90 |
| 16 | 91,32 % | 8,68 % | - | - | - | - | - | 25,80 | 86,00 |

Tabelle 10: Zusammensetzung und Auswaage des Kondensats der Beispiele 15 - 16 für die Versuchszeit von 0 - 2 h.

EP 0 496 206 B1

| Beispiel | Benzol | Chlor-benzol | 1,2-Di-chlor-benzol | 1,3-Di-chlor-benzol | 1,4-Di-chlor-benzol | 1,2,3-Tri-chlorbenzol | 1,2,4-Tri-chlorbenzol | Auswaage g | Auswaage % Einsatz |
|---|---|---|---|---|---|---|---|---|---|
| 15 | 84,73 % | 13,79 % | 1,24 | 0,19 | 0,05 | - | - | 28,86 | 96,20 |
| 16 | 93,38 % | 6,62 % | - | - | - | - | - | 25,73 | 85,77 |

Tabelle 11: Zusammensetzung und Auswaage des Kondensats der Beispiele 15 - 16 für die Versuchszeit von 2 - 4 h.

EP 0 496 206 B1

EP 0 496 206 B1

| Beispiel | Benzol | Chlor-benzol | 1,2-Di-chlor-benzol | 1,3-Di-chlor-benzol | 1,4-Di-chlor-benzol | 1,2,3-Tri-chlorbenzol | 1,2,4-Tri-chlorbenzol | Auswaage g | Auswaage % Einsatz |
|---|---|---|---|---|---|---|---|---|---|
| 15 | 82,07 % | 11,24 % | 4,91 | 0,98 | 0,80 | - | - | 29,50 | 98,33 |
| 16 | 93,12 % | 5,62 % | 0,62 | 0,22 | 0,22 | - | - | 26,47 | 88,23 |

Tabelle 12: Zusammensetzung und Auswaage des Kondensats der Beispiele 15 - 16 für die Versuchszeit von 4 - 7 h (Ende der Nachlaufzeit).

**Patentansprüche**

1. Verfahren zur Umsetzung von polyhalogenierten Aromaten mit monohalogenierten oder nichthalogenierten Aromaten, bei dem das Halogen der polyhalogenierten Aromaten auf die monohalogenierten oder

19

nicht halogenierten Aromaten und gleichzeitig der Wasserstoff der monohalogenierten oder nicht halogenierten Aromaten auf die polyhalogenierten Aromaten in Gegenwart eines Katalysators übertragen wird, dadurch gekennzeicht, daß man einen Katalysator einsetzt, der Ruthenium als Element oder in Form von Verbindungen und gegebenenfalls ein oder mehrere Metalle oder Metallverbindungen aus der I. und II. Nebengruppe des Periodensystems enthält.

2. Verfahren nach Anspruch 1, dadurch gekennzeichnet, daß man als polyhalogenierten Aromaten 2 bis 6-fach halogeniertes Benzol, 2 bis 8-fach halogeniertes Naphthalin oder 2 bis 10-fach halogeniertes Biphenyl einsetzt, wobei Halogen Chlor, Brom oder Jod bedeuten kann.

3. Verfahren nach Anspruch 1, dadurch gekennzeichnet, daß man als monohalogenierten oder nicht halognierten Aromaten Fluorbenzol, Chlorbenzol, Brombenzol, Jodbenzol oder Benzol einsetzt.

4. Verfahren nach Anspruch 1, dadurch gekennzeichnet, daß man als Katalysator eine mit Rutheniumverbindungen beladene Aktivkohle einsetzt.

5. Verfahren nach Anspruch 4, dadurch gekennzeichnet, daß man als Rutheniumverbindung Rutheniumchlorid einsetzt.

6. Verfahren nach Anspruch 1, dadurch gekennzeichnet, daß man als Katalysator eine mit Rutheniumverbindungen und zusätzlich mit Verbindungen der I. und II. Nebengruppe des Periodensystems beladene Aktivkohle einsetzt.

7. Verfahren nach Anspruch 6, dadurch gekennzeichnet, daß die Verbindungen der I. und II. Nebengruppe Verbindungen von Kupfer, Silber und Zink bedeuten.

8. Verfahren nach Anspruch 6, dadurch gekennzeichnet, daß man als Katalysator eine mit Rutheniumchlorid und einer Silberverbindung beladene Aktivkohle als Katalysator einsetzt.

9. Verfahren nach Anspruch 6, dadurch gekennzeichnet, daß man als Katalysator eine mit Rutheniumchlorid und einer Kupferverbindung beladene Aktivkohle als Katalysator einsetzt.

10. Verfahren nach Anspruch 1, dadurch gekennzeichnet, daß man bei einer Temperatur von 200°C bis 600°C arbeitet.

## Claims

1. Process for reacting polyhalogenated aromatic compounds with monohalogenated or nonhalogenated aromatic compounds, in which the halogen of the polyhalogenated aromatic compounds is transferred to the monohalogenated or nonhalogenated aromatic compounds and the hydrogen of the monohalogenated or nonhalogenated aromatic compounds is simultaneously transferred to the polyhalogenated aromatic compounds in the presence of a catalyst, characterised in that a catalyst is used which contains ruthenium as the element or in the form of compounds and if desired one or more metals or compounds of metals from subgroups I and II of the Periodic Table.

2. Process according to Claim 1, characterised in that the polyhalogenated aromatic compound used is di- to hexahalogenated benzene, di- to octahalogenated naphthalene or di- to decahalogenated biphenyl, where halogen can be chlorine, bromine or iodine.

3. Process according to Claim 1, characterised in that the monohalogenated or nonhalogenated aromatic compound used is fluorobenzene, chlorobenzene, bromobenzene, iodobenzene or benzene.

4. Process according to Claim 1, characterised in that the catalyst used is an activated charcoal charged with ruthenium compounds.

5. Process according to Claim 4, characterised in that the ruthenium compound used is ruthenium chloride.

6. Process according to Claim 1, characterised in that the catalyst used is an activated charcoal charged with ruthenium compounds and additionally with compounds from subgroups I and II of the Periodic Table.

7. Process according to Claim 6, characterised in that the compounds from subgroups I and II are compounds of copper, silver and zinc.

8. Process according to Claim 6, characterised in that the catalyst used is an activated charcoal charged with ruth+enium chloride and a silver compound.

9. Process according to Claim 6, characterised in that the catalyst used is an activated charcoal charged with ruthenium chloride and a copper compound.

10. Process according to Claim 1, characterised in that a temperature of 200°C to 600°C is employed.

**Revendications**

1. Procédé de mise à réagir de composés aromatiques polyhalogénés avec des composés aromatiques monohalogénés ou non halogénés, dans lequel l'halogène des composés aromatiques polyhalogénés est transféré aux composés aromatiques monohalogénés ou non halogénés et l'hydrogène des composés aromatiques monohalogénés ou non halogénés est transféré aux composés aromatiques polyhalogénés en présence d'un catalyseur, caractérisé en ce qu'on utilise un catalyseur qui contient du ruthénium sous forme d'élément ou de composés et le cas échéant un ou plusieurs métaux ou composés métalliques des sous-groupes IB et IIB de la Classification Périodique des Eléments.

2. Procédé selon la revendication 1, caractérisé en ce qu'on utilise comme composés aromatiques polyhalogénés du benzène deux à six fois halogéné, du naphtalène deux à huit fois halogéné ou du biphényle deux à dix fois halogéné, l'halogène pouvant être le chlore, le brome ou l'iode.

3. Procédé selon la revendication 1, caractérisé en ce qu'on utilise comme composés aromatiques monohalogénés ou non halogénés du fluorobenzène, du chlorobenzène, du bromobenzène, de l'iodobenzène ou du benzène.

4. Procédé selon la revendication 1, caractérisé en ce qu'on utilise comme catalyseur un charbon actif chargé de composés de ruthénium.

5. Procédé selon la revendication 4, caractérisé en ce qu'on utilise comme composés de ruthénium du chlorure de ruthénium.

6. Procédé selon la revendication 1, caractérisé en ce qu'on utilise comme catalyseur un charbon actif chargé de composés de ruthénium et additionnellement de composés des sous-groupes IB et IIB de la Classification Périodique des Eléments.

7. Procédé selon la revendication 6, caractérisé en ce que les composés des sous-groupes IB et IIB désignent des composés de cuivre, argent et zinc.

8. Procédé selon la revendication 6, caractérisé en ce qu'on utilise comme catalyseur un charbon actif chargé de chlorure de ruthénium et d'un composé d'argent.

9. Procédé selon la revendication 6, caractérisé en ce qu'on utilise comme catalyseur un charbon actif chargé de chlorure de ruthénium et d'un composé de cuivre.

10. Procédé selon la revendication 1, caractérisé en ce qu'on opère à une température comprise entre 200°C et 600°C.